# EUROPÄISCHE PATENTSCHRIFT

(11) **EP 3 165 196 B1**
(45) Veröffentlichungstag und Bekanntmachung des Hinweises auf die Patenterteilung: **27.10.2021**
(21) Anmeldenummer: 16197108.0
(22) Anmeldetag: 03.11.2016
(51) Int. Cl.: A61F 2/07, A61F 2/958, A61F 2/00, A61F 2/966, A61M 25/10, A61F 2/04, A61F 2/92, A61M 25/00

(54) **VORRICHTUNG ZUR EINBRINGUNG EINES GEWEBEERSATZES IN EIN HOHLORGAN**
DEVICE FOR INTRODUCING A TISSUE SUBSTITUTE INTO A HOLLOW ORGAN
DISPOSITIF D'INTRODUCTION D'UN REMPLACEMENT DE TISSU DANS UN ORGANE CREUX

(30) Priorität: 03.11.2015 DE 102015118854
(43) Veröffentlichungstag der Anmeldung: 10.05.2017
(73) Patentinhaber: Mukocell GmbH, 44227 Dortmund (DE)
(72) Erfinder: Liebig, Soeren, 41468 Neuss (DE); Ram-Liebig, Gouya, 41468 Neuss (DE); Liebig, Andian, 41468 Neuss (DE)
(74) Vertreter: Riechelmann & Carlsohn Patentanwälte PartG mbB

(56) Entgegenhaltungen:
- DE-A1- 10 060 443
- US-A1- 2003 236 565
- US-A1- 2005 222 661
- US-A1- 2006 074 478
- US-A1- 2011 238 167
- US-A1- 2014 094 751

## Beschreibung

Die Erfindung betrifft eine Vorrichtung zur Einbringung eines Gewebeersatzes in ein Hohlorgan, insbesondere in eine Harnröhre. Sie betrifft ferner ein Verfahren zur Herstellung einer solchen Vorrichtung. Die erfindungsgemäße Vorrichtung ist insbesondere zur Behandlung von Harnröhrenstrikturen geeignet.

Gefäßstrukturen und andere Organe, die die Leitung verschiedener Substanzen innerhalb des menschlichen oder tierischen Körpers ermöglichen sollen, können durch Strikturen, Stenosen und andere Obstruktionen beeinträchtigt werden. Derartige Gefäße und Organe umfassen insbesondere Blutgefäße, Lymphgefäße, die Speiseröhre, den Magen-Darm-Trakt, die Gallenblase, die Luftröhre, das Herz, das Nierenbecken, den Harnleiter, die Harnblase, die Harnröhre und die Eileiter, wobei die Aufzählung nicht vollständig ist. Die Gefäßstrukturen und Organe umschließen dabei einen Hohlraum, der als Lumen bezeichnet wird. Die Organe werden deshalb auch als Hohlorgane bezeichnet. In der Fachsprache wird nicht streng zwischen Strikturen und Stenosen unterschieden. Gelegentlich werden akut aufgetretene entzündliche Veränderungen als Stenose bezeichnet, während chronische Veränderungen, die eine Spätfolge eines chronischen Entzündungsprozesses sind, als Strikturen bezeichnet werden. In den meisten Fällen bezeichnen beide Begriffe ein- und denselben Sachverhalt, nämlich die Verengung des Lumens eines Gefäßes oder Hohlorgans.

Verengungen der Harnröhre, die auch als Harnröhrenstrikturen oder Urethrastrikturen bezeichnet werden, können insbesondere durch Infektionen der Harnröhre, eine sogenannte Urethritis, Tumore oder direkte Verletzungen verursacht werden. Auch Blasenkatheter können zu einer Harnröhrenstriktur führen. Folge einer solchen Verengung ist ein abgeschwächter Harnstrahl oder in schweren Fällen ein Harnverhalt. In beiden Fällen kann die Harnblase nicht mehr vollständig entleert werden, was wiederum zu einer Harnblasenentzündung führen kann. Ferner kann eine Harnröhrenstriktur Komplikationen wie Nierenschäden, Nierensteinerkrankung und sexuelle Störungen bedingen. Eine Verengung der Harnröhre kann auch angeboren sein.

Zur Behandlung von Verengungen der Harnröhre wird typischerweise die Urethrotomie (Harnröhrenschlitzung) eingesetzt, bei der die Harnröhre an dem verengten Abschnitt in der Regel von innen mit einem Messer oder Laser längs geschlitzt wird. Anschließend wird ein Blasenkatheter gelegt, der einige Tage in der Harnröhre verbleibt. Allein in Deutschland werden ca. 45.000 Schlitzungen pro Jahr durchgeführt, wobei viele Patienten mehrfach behandelt werden müssen. Das Verfahren wird endoskopisch durchgeführt, benötigt somit keine offene Operation und ist dadurch risikoarm. Zusätzlich sind die Operations- und Narkosezeit relativ kurz. Diese Behandlungsmethode ist somit in Relation zu den anderen Behandlungsmethoden pro Eingriff kostengünstiger. Die 12-Monats-Fehlerrate liegt jedoch hierbei bei 70 %, bei wiederholten Schlitzungen sinken die Heilungschancen sogar auf bis zu 0 %. Nach jeder Behandlungswiederholung erhöht sich die Wahrscheinlichkeit einer Restriktur bzw. Restenose. Ein Patient muss somit über seinen Lebenszeitraum mehrfach (teilweise zwei bis drei Mal pro Jahr) behandelt werden. Aus diesem Grund gilt die nur temporär einzusetzende Urethrotomie nicht als eine sinnvolle Methode zur Behandlung von Harnröhrenstrikturen.

Im Gegensatz zur bloßen Urethrotomie wird bei einer Harnröhren-Rekonstruktion oder Harnröhren-Plastik (Urethroplastie) mit nativen Mundschleimhaut-Segmenten eine akzeptable Fehlerrate von ca. 30 % erreicht. Im Gegensatz zur Harnröhrenschlitzung muss die Harnröhre für die Gewebeeinbringung und die Rekonstruktion geöffnet werden. Nach der Eröffnung der Haut-, Unterhaut- und Muskelgewebe wird die Harnröhre mit Schere oder Messer geöffnet. Anschließend wird ein Abschnitt der Mundschleimhaut in die geöffnete Harnröhre aufgebracht und vernäht. Danach wird ein Blasenkatheter zur Schienung und Offenhaltung der Harnröhre gelegt. Ferner ist ein Bauchdeckenkatheter erforderlich, der in die Blase eingelegt wird, um diese entleeren zu können. Der Nachteil dieser Rekonstruktionstechnik (oder Plastik) ist die Notwendigkeit einer offenen Operation und die damit verbundenen Komplikationen (wie z. B. Blutung, Wundheilungsstörung, Lange Operationszeit und Anästhesie, lange postoperative Krankenhausaufenthalt, Thrombose, Embolie, und weitere. Die meist großflächige Entnahme von Mundschleimhaut birgt allerdings damit verbundene Komplikationen wie Schmerzen, Blutungen oder Vernarbungen mit möglichen Folgen für Mimik, Nahrungsaufnahme und Beweglichkeit der Unterlippe. Aktuelle Statistiken weisen bei 18 bis 30 % der Patienten Langzeitkomplikationen durch die Entnahme von Mundschleimhaut auf. Auch ist die Entnahme von nativer Mundschleimhaut bei Patienten nur in begrenztem Umfang möglich. Eine Harnröhren-Plastik, bei der mittels Tissue-Engineering im Labor gezüchtete Patientenzellen (wie z. B. Patientenzellen, die unter der Bezeichnung MukoCell kommerziell erhältlich sind), eingesetzt werden, ist schon seit einigen Jahren bei Patienten mit Harnröhrenstriktur in klinischer Anwendung (Ram-Liebig et al., Adv Drug Deliv Rev. 2015 Mar; 82-83: 181-91, Regulatory challenges for autologous tissue engineered products on their way from bench to bedside in Europe). Dieses Verfahren benötigt zwar auch eine offene Harnröhrenoperation, vermeidet jedoch eine großflächige Entnahme der nativen Mundschleimhaut, da eine nur kleine Biopsie für die Herstellung des Tissue-Engineering-Produkte meist ausreichend ist. Prinzipiell kann man aber sagen, dass das Aufbringen von Ersatzgewebe (ob natives Gewebe oder Tissue-Engineering Gewebe) die Erfolgsrate einer Harnröhrenoperation langfristig deutlich erhöht.

Verengungen des Harnleiters, die auch als Ureterstrikturen bezeichnet werden, können zu funktionalen Beeinträchtigungen, insbesondere Ablassstörungen, führen. Die Strikturen können durch Erkrankungen verursacht werden, sie können jedoch auch die Folge einer endoskopischen Entfernung von Nieren- und Harnsteinen sein. Harnleiterstrikturen entstehen häufig durch eine Ischämie oder Einschränkung der Blutversorgung. Sie können aber auch angeboren sein. Harnleiterstrikturen können ebenfalls mit Ersatzgewebe wie Mundschleimhaut behandelt werden.

Eine denkbare Alternative, um erneute Verengungen des Lumens, sogenannte Restenosen, zu verhindern, wäre die Einbringung von Stents, Kathetern oder Schienen in den Lumen. Solche Platzhalter bestehen zumeist aus biokompatiblen Metallen oder Metalllegierungen oder Silikon. Stents können netzartig aufgebaut sein, um ein Einwachsen des Gewebes in den Stent zu erleichtern. Es hat sich jedoch herausgestellt, dass derartige Stents eine ungesteuerte Entwicklung des umgebenden Gewebes verursachen oder ihr zumindest ausgesetzt sein können. In den urologischen Organen ist der Platzhalter zusätzlich dem aggressiven Urin dauerhaft ausgesetzt. Es kommt somit sehr schnell zur Enkrustationen, Infektionen und Abstoßungen. Daher müssen Stents, Katheter oder Schienen, die zurzeit klinisch Anwendung finden und in die in urologische Hohlorgane eingelegt werden, regelmäßig (meist nach 6 Wochen) getauscht werden. Somit präsentieren Platzhalter bis zum heutigen Tag nur eine vorübergehende Lösung für eine Vermeidung der Restenosierung einer urologischen Verengung.

Eine denkbare weitere Lösung wäre die Implantation von Grafts, deren Oberfläche mit Zellen oder Wachstumsfaktoren bzw. weiteren strukturellen Änderungen so verarbeitet wurde, dass eine gesunde Wundheilung nach der Implantation des Grafts induziert wird und somit eine Restenosierung verhindert wird. Dies kann entweder durch lebende Zellen erreicht werden, die ungeschädigt in das röhrenförmige Organ implantiert werden und Wachstumsfaktoren sezernieren, oder aber durch weitere Optionen wie Beschichtung der Graftoberfläche mit Wachstumsfaktoren sowie strukturellen Änderung der Graftoberfläche.

Es sind zurzeit als Behandlungsmethoden entweder eine endoskopische (minimal-invasive und risikoarme) Schlitzung mit jedoch niedrigerer Erfolgsrate oder eine offene (invasive und risikoreiche) Rekonstruktion mit Ersatzgewebe mit hoher Erfolgsrate möglich.

Aus US 2003/236565 A1 ist eine Vorrichtung zum Einbringen eines expandierbaren Implantates in ein Lumen bekannt.

Aufgabe der Erfindung ist es, die Nachteile nach dem Stand der Technik zu beseitigen. Es sollen insbesondere eine Vorrichtung und eine Anordnung zur endoskopischen Einführung eines Gewebeersatzes in ein Hohlorgan angegeben werden, die eine minimal-invasive, risikoarme, medizinisch sichere, eine hohe Erfolgsrate sichernde und kostengünstigere Behandlung von Verengungen von Gefäßen und Hohlorganen erlauben.

Diese Aufgabe wird durch die Merkmale der Ansprüche 1 und 9 gelöst. Zweckmäßige Ausgestaltungen der Erfindungen ergeben sich aus den Merkmalen der Unteransprüche.

Nach Maßgabe der Erfindung ist eine Vorrichtung zum Einbringen eines Gewebeersatzes in ein Hohlorgan vorgesehen. Die Vorrichtung umfasst einen Träger mit einer Oberfläche zum Tragen des Gewebeersatzes und ein Schutzelement zum Schutz des Gewebeersatzes während des Einbringens der Vorrichtung in das Hohlorgan. die Vorrichtung umfasst:
- einen als Schutzelement dienenden ersten Tubus mit einem offenen proximalen Ende;
- einen als Träger dienenden zweiten Tubus, der in den ersten Tubus einführbar ist und einen durch Zufuhr eines Fluids ersten expandierbaren Abschnitt mit der Oberfläche zum Tragen des Gewebeersatzes aufweist; und
- einen dritten Tubus, der durch den zweiten Tubus und das offene proximale Ende des ersten Tubus führbar ist, wobei der dritte Tubus an seinem proximalen Ende einen durch Zu-fuhr eines Fluids zweiten expandierbaren Abschnitt aufweist.

Die erfindungsgemäße Vorrichtung ermöglicht das Einbringen eines Gewebeersatzes in ein Hohlorgan, ohne diesen Gewebeersatz zu verletzen, insbesondere ohne dass es zu einer mechanischen Reibung und dadurch zu einer Schädigung oder Verletzung des Gewebeersatzes während der Einbringung kommt.

Die erfindungsgemäße Vorrichtung kann nach Inkorporierung des Gewebeersatzes in das Wundbett am Zielort, meist nach vier bis sechs Wochen, entfernt werden, wobei der Gewebeersatz am Zielort verbleibt.. Die erfindungsgemäße Vorrichtung erlaubt das endoskopische Aufbringen eines Gewebeersatzes in einen Hohlorgan wie die Harnröhre, wobei die Zellen des Gewebeersatzes während des Vorschiebens in dem Lumen des Hohlorgans geschützt und nicht verletzt werden. Zusätzlich erlaubt diese Vorrichtung das Fixieren des Gewebeersatzes an das gut durchblutete Wundbett am Zielort. Dieser Schritt ist vorteilhaft für eine Inkorporierung des Gewebeersatzes in das Wundbett am Zielort und somit für eine gesunde und schnelle Wundheilung. Die erfindungsgemäße Vorrichtung kann nach Inkorporierung des Gewebeersatzes problemlos entfernt werden, wobei der Gewebeersatz am Zielort verbleibt. Es wird somit eine Enkrustration, Infektion oder Abstoßung der erfindungsgemäßen Vorrichtung vermieden.

Die erfindungsgemäße Vorrichtung erlaubt die Rekonstruktion eines Hohlorgans durch das Aufbringen eines Gewebeersatzes in einem endoskopischen, nicht-offenen und somit minimal-invasiven Eingriff. Somit kann eine risikoarme minimal-invasive Therapieform mit einer hohen Erfolgsrate erreicht werden.

Bei dem Gewebeersatz kann es sich um ein Implantat oder Transplantat, das in der Fachsprache auch als Graft bezeichnet wird, handeln. Die erfindungsgemäße Vorrichtung ermöglicht es insbesondere, den Gewebeersatz unbeschädigt in ein Hohlorgan, insbesondere ein röhrchenförmiges Organ, einzubringen, um dort eine Geweberegeneration zu induzieren. Die erfindungsgemäße Vorrichtung ist insbesondere für die Rekonstruktion von Hohlorganen geeignet. Bei dem Hohlorgan kann es sich beispielsweise um die Speiseröhre, den Magen-Darm-Trakt, die Gallenblase, die Luftröhre, das Herz, das Nierenbecken, den Harnleiter, die Harnblase, die Harnröhre und den Eileiter handeln, wobei die Aufzählung nicht vollständig ist. Das Hohlorgan ist vorzugsweise die Harnröhre oder der Harnleiter. Bei dem Hohlorgan kann es sich aber auch um ein Gefäß, beispielsweise ein Blutgefäß oder ein Lymphgefäß handeln. Bei dem Hohlorgan handelt es sich insbesondere um ein Hohlorgan eines Menschen oder Tieres.

Vorzugsweise ist der Gewebeersatz aus der Gruppe ausgewählt, die Haut, natives Gewebe, künstlich hergestelltes Gewebe, eine Membran und einen Membranverbund umfasst. Bei dem Gewebeersatz kann es sich um ein oder mehrere Gewebe-Segmente, insbesondere ein oder mehrere native Gewebe-Segmente handeln.

Die Membran oder der Membranverbund können mit Zellen besiedelt sein, was jedoch nicht erforderlich ist. Soll die Vorrichtung zur Einbringung eines Gewebeersatzes in ein Harnorgan verwendet werden, so handelt es sich bei den Zellen vorzugsweise um Mukosagewebezellen.

Mittels der erfindungsgemäßen Vorrichtung wird der Gewebeersatz in dem Lumen zum Zielort geführt und dort für einen vorgegebenen Zeitraum gehalten. Ein Vernähen des Gewebeersatzes an den die Schlitzung umgebenden Bereichen des Hohlorgans ist nicht länger erforderlich. Im Falle einer Harnröhre sorgen beispielsweise Zellen des Gewebeersatzes, wie Mukosagewebezellen, für ein derart rasches Einwachsen von Gewebe in den Gewebeersatz, dass eine Fixierung des Gewebeersatzes am Hohlorgan nicht erforderlich ist. Sie kann dennoch vorgenommen werden.

Bei der Haut kann es sich um native Haut handeln. Ein Beispiel einer geeigneten Haut ist eine Mundschleimhaut, beispielsweise eine native Mundschleimhaut. Unter künstlich hergestelltem Gewebe wird insbesondere Gewebe verstanden, das im Labor mittels Techniken des Tissue Engineering hergestellt wird.

Bei der Membran handelt es sich vorzugsweise um eine biologisch kompatible Membran, z. B. eine Membran aus Kollagen. Beispiele einer geeigneten Membran sind eine Membran, die Zellen und/oder Zellbestandteile aufweist; eine Membran, die keine Zellen und Zellbestandteile aufweist; eine Membran mit behandelter Oberfläche sowie eine Membran mit unbehandelter Oberfläche. Eine Membran mit behandelter Oberfläche ist beispielsweise eine Membran, auf deren Oberfläche Zellen und/oder Zellbestandteile aufgebracht oder deren Oberfläche strukturellen Änderungen unterzogen wurden. Die Zellen und/oder Zellbestandteile können auf die Oberfläche der Membran unter Ausbildung von Schichten aufgebracht sein, wodurch eine beschichtete Membran erhalten wird. Bei den Zellen handelt es sich vorzugsweise um autologe Zellen. Bei den Zellen kann es sich um Mukosagewebezellen, beispielsweise um Mundschleimhautzellen handeln. Bei den Zellbestandteilen handelt es sich beispielsweise um Wachstumsfaktoren.

Membranen, die Zellen oder Zellbestandteile aufweisen, Membranen, auf deren Oberfläche Zellen oder Wachstumsfaktoren aufgebracht sind, sowie Membranen, deren Oberfläche strukturellen Änderungen unterzogen wurde, bieten den Vorteil, dass nach einer Implantation einer solchen Membran die umgebenden Zellen des Hohlorgans von der Membran angezogen werden und auf dieser wachsen. Damit wird eine Regeneration des Hohlorgans induziert und beschleunigt. Es ist daher von besonderer Bedeutung, dass die Oberfläche des Gewebeersatzes nicht beschädigt wird, wenn der Gewebeersatz in das Hohlorgan eingeführt wird. Das gilt insbesondere auch dann, wenn die Oberfläche des Gewebeersatzes Zellen, z. B. Mukosagewebezellen wie Mundschleimhautzellen, trägt.

Der Membranverbund kann eine oder mehrere derartige Membranen umfassen. Beispiele eines bevorzugten Gewebeersatzes sind eine Membran, die mit Mukosagewebezellen besiedelt ist, oder ein Membranverbund, der mit Mukosagewebezellen besiedelt ist.

Weitere Einzelheiten zur Membran, dem Membranverbund und den Mukosagewebezellen können den nachstehenden Abschnitten "Membran", "Membranverbund" und "Mukosagewebezellen" entnommen werden.

Bei dem Träger kann es sich um einen rohrförmigen Körper handeln. Bei dem Träger handelt es sich um einen Tubus. Bei dem Tubus kann es sich um einen Katheter, beispielsweise um einen Ballonkatheter, handeln. Der Träger weist eine Oberfläche auf, auf die der Gewebeersatz aufgebracht werden kann, um ihn mittels der erfindungsgemäßen Vorrichtung zu einen Ort, den Zielort, innerhalb des Lumens eines Hohlorganes zu transportieren, und zwar ohne dabei den Gewebeersatz zu beschädigen. Am Zielort wird dann das Schutzelement entfernt. Der Gewebeersatz kann dann von der Oberfläche abgelöst werden, auch wenn das nicht zwingend erforderlich ist. Die Oberfläche des Trägers, auf die der Gewebeersatz aufgebracht werden kann, ist die erfindungsgemäß vorgesehene Oberfläche zum Tragen des Gewebeersatzes. Die Gewebeersatz-Trägeroberfläche, auf die der Gewebeersatz aufgebracht ist, wird im Folgenden als beschichtete Gewebeersatz-Trägeroberfläche bezeichnet. Bei dem Zielort kann es sich beispielsweise um den Ort der Verengung, der Striktur, der Schlitzung oder einen anderen vom Operateur vorgesehenen Ort innerhalb des Hohlorgans handeln. Die erfindungsgemäße Vorrichtung hat den Zielort erreicht, wenn die beschichtete Gewebeersatz-Trägeroberfläche der Verengung, der Striktur oder der Schlitzung gegenüberliegt, genauer den Bereich der Innenseite des Hohlorgans, in dem sich die Verengung, Striktur oder Schlitzung befindet.

Die Größe der Gewebeersatz-Trägeroberfläche hängt von der Größe des Gewebeersatzes ab, der an der Gewebeersatz-Trägeroberfläche befestigt werden soll. Vorzugsweise hat die Gewebeersatz-Trägeroberfläche eine Länge von 3 bis 12 cm, stärker bevorzugt von 5 bis 10 cm, am stärksten bevorzugt von 8 cm. Die Länge der Gewebeersatz-Trägeroberfläche bezieht sich dabei auf deren Ausdehnung parallel zur Längsachse des Trägers. Die Ausdehnung des ersten expandierbaren Abschnittes in dieser Richtung kann gleich oder größer als die Länge der Gewebeersatz-Trägeroberfläche sein. In Umfangsrichtung kann die Ausdehnung der Gewebeersatz-Trägeroberfläche der Breite oder dem Umfang des Trägers oder einem Teil der Breite oder des Umfangs des Trägers entsprechen.
Auf der Gewebeersatz-Trägeroberfläche können sich regelmäßig oder unregelmäßig angeordnete Erhöhungen, beispielsweise Noppen, oder Vertiefungen befinden, um die Haftung des Gewebeersatzes darauf zu verbessern. Beispielsweise kann die Gewebeersatz-Trägeroberfläche Riffelungen aufweisen. Die Gewebeersatz-Trägeroberfläche kann alternativ oder zusätzlich angeraut sein. In einer bevorzugten Ausführungsform ist die Gewebeersatz-Trägeroberfläche angeraut und noppenförmig.

Der Gewebeersatz muss nicht die gesamte Gewebeersatz-Trägeroberfläche bedecken, auch wenn dies möglich ist. Es kann vielmehr vorgesehen sein, dass der Gewebeersatz nur eine Teilfläche der Gewebeersatz-Trägeroberfläche bedeckt. Die Teilfläche muss dabei so bemessen sein, dass der Gewebeersatz die gesamte Schlitzung, die gesamte Striktur oder den gesamten verengten Abschnitt bedecken kann. Vorzugsweise ist der Gewebeersatz ein flächiges Gebilde.

Weitere Einzelheiten zum Träger können dem nachfolgenden Abschnitt "Tubus als Träger" entnommen werden.

Das Schutzelement ist ein Tubus oder eine Kombination aus einem Tubus und einer Schutzhülle oder einem oder mehreren Schutzdrähten. Das Schutzelement soll verhindern, dass der Gewebeersatz, wenn die erfindungsgemäße Vorrichtung durch eine Öffnung in den Lumen eines Gefäßes oder Hohlorgans eingeführt wird, beschädigt wird, bevor er den Zielort in dem Gefäß oder Hohlorgan erreicht, beispielsweise durch abrasiven Kontakt mit der Innenwand des Gefäßes oder Hohlorgans. Das Schutzelement muss entfernt werden, sobald die erfindungsgemäße Vorrichtung den Zielort erreicht hat.

Das Schutzelement kann daher lösbar an einem anderen Element der erfindungsgemäßen Vorrichtung befestigt werden, beispielsweise an dem Gewebeersatz oder an dem Träger. Alternativ kann das Schutzelement über eine erste Stirnseite des Trägers geführt sein, und zwar die Stirnseite des Trägers, die zuerst in das Lumen eingeführt wird. Nachdem die erfindungsgemäße Vorrichtung den Zielort erreicht hat, kann das Schutzelement nach hinten, also in entgegengesetzter Richtung zur Einführungsrichtung der erfindungsgemäßen Vorrichtung in den Lumen, von dem Träger oder dem Gewebeersatz abgezogen und aus dem Gefäß oder Hohlorgan entfernt werden.

Der Gewebeersatz ist zweckmäßigerweise zwischen dem Träger und dem Schutzelement angeordnet. Die Schutzhülle kann beispielsweise eine Folie aus einem biologisch kompatiblen Material sein. Zweckmäßigerweise ist die Folie ein schlauchförmiges Gebilde, das die übrigen Bestandteile der erfindungsgemäßen Vorrichtung aufnimmt und das an der ersten Stirnseite des Trägers geschlossen ist. Wird die Folie an der ersten Stirnseite, beispielsweise mittels eines Messers, das durch einen rohrförmigen Träger geführt wird, zerstört, so kann sie dann aus dem Lumen entfernt werden, wodurch der Gewebeersatz in Kontakt mit der Innenwand des Gefäßes oder Hohlorgans gelangt.

Sind Schutzdrähte vorgesehen, so verlaufen diese im Wesentlichen parallel zur Längsachse des Trägers, wobei der Abstand zwischen den Drähten so bemessen ist, dass eine Beschädigung des Gewebeersatzes während der Einführung in das Gefäß oder Hohlorgan verhindert wird. Beispielsweise können vier oder mehr Drähte vorgesehen sein, die gleichmäßig voneinander beabstandet sind und deren radialer Abstand von der Längsachse des Trägers der gleiche ist.

Die erfindungsgemäße Vorrichtung kann Befestigungsmittel umfassen, mit denen der Gewebeersatz an der Gewebeersatz-Trägeroberfläche lösbar befestigt werden kann. Die Befestigungsmittel ermöglichen es, den Gewebeersatz von der Gewebeersatz-Trägeroberfläche zu trennen. Bei dem Befestigungsmittel kann es sich beispielsweise um eine oder mehrere Klemmen, Klammern, Clips oder Kombinationen davon handeln.

### Tubus als Träger

Die erfindungsgemäße Vorrichtung umfasst:
- einen ersten, als Schutzelement dienenden Tubus mit einem offenen proximalen Ende; und
- einen zweiten, als Träger dienenden Tubus, der in den ersten Tubus einführbar ist und einen durch Zufuhr eines Fluids ersten expandierbaren Abschnitt mit einer Oberfläche zur Aufnahme des Gewebeersatzes aufweist.

Zusätzlich zu dem ersten und zweiten Tubus umfasst die erfindungsgemäße Vorrichtung:
- einen dritten Tubus, der durch den zweiten Tubus und durch das offene proximale Ende des ersten Tubus führbar ist, wobei der dritte Tubus an seinem proximalen Ende einen durch Zufuhr eines Fluids expandierbaren zweiten Abschnitt aufweist.

Die Oberfläche zum Tragen des Gewebeersatzes, die an dem ersten expandierten Abschnitt des zweiten Tubus ausgebildet ist, ist die Gewebeersatz-Trägeroberfläche. Auf die Gewebeersatz-Trägeroberfläche des zweiten Tubus wird der Gewebeersatz aufgebracht, um ihn in einem Hohlorgan an einen Zielort zu transportieren.

Mittels dieser Ausführungsform der erfindungsgemäßen Vorrichtung ist es möglich, einen Gewebeersatz durch das Hohlorgan an den Zielort, beispielsweise den Ort der Verengung, der Schlitzung oder Striktur zu transportieren.

Dazu kann folgendermaßen vorgegangen werden: Der Gewebeersatz wird auf die Gewebeersatz-Trägeroberfläche unter Erhalt der beschichteten Gewebeersatz-Trägeroberfläche aufgebracht. Dazu wird der zweite Tubus aus dem ersten Tubus entnommen, wodurch die Gewebeersatz-Trägeroberfläche zugänglich wird. Nach dem Aufbringen des Gewebeersatzes - wodurch die Gewebeersatz-Trägeroberfläche in die beschichtete Gewebeersatz-Trägeroberfläche überführt wird - wird der zweite Tubus in den ersten Tubus eingeführt. Um das zu ermöglichen, weist der zweite Tubus einen Außendurchmesser auf, der geringer als der Innendurchmesser des ersten Tubus ist. Der Abstand zwischen der Außenseite des zweiten Tubus und der Innenseite des ersten Tubus kann dabei so ausgebildet sein, dass der Gewebeersatz die Innenseite des ersten Tubus nicht berührt. Die Längsachse des zweiten Tubus ist koaxial zur Längsachse des ersten Tubus.

Der erste Tubus schützt die beschichtete Gewebeersatz-Trägeroberfläche und damit den Gewebeersatz gegen eine Beschädigung beim Einführen der erfindungsgemäßen Vorrichtung in das Hohlorgan. Die Außenseite des ersten Tubus bildet die Außenseite dieser Variante der erfindungsgemäßen Vorrichtung. Der erste Tubus weist ein offenes proximales Ende auf. In einer Ausführungsform der Erfindung ist der zweite Tubus bis zum offenen proximalen Ende des ersten Tubus geführt, ohne jedoch durch das offene Ende geführt zu sein. Vielmehr soll das offene proximale Ende des ersten Tubus den Durchtritt des dritten Tubus ermöglichen.

Der dritte Tubus ist durch den zweiten Tubus und durch das offene proximale Ende des ersten Tubus geführt. An seinem proximalen Ende weist er einen durch Zufuhr eines Fluids zweiten expandierbaren Abschnitt auf. Die Längsachse des dritten Tubus ist vorzugsweise koaxial zur Längsachse des zweiten Tubus angeordnet. Der zweite expandierbare Abschnitt ist vorzugsweise außerhalb des ersten Tubus angeordnet. Der zweite expandierbare Abschnitt bildet somit das proximale Ende dieser Variante der erfindungsgemäßen Vorrichtung.

Der zweite expandierbare Abschnitt weist eine maximale Ausdehnung quer zur Längsachse des dritten Tubus auf, die der Ausdehnung des ersten Tubus, quer zu dessen Längsachse, entspricht oder dessen Ausdehnung übersteigt. Der zweite expandierbare Abschnitt besitzt seine maximale Ausdehnung im expandierten Zustand. Vorzugsweise bildet der zweite expandierbare Abschnitt im expandierten Zustand eine Spitze, deren Außendurchmesser sich zum proximalen Ende der Vorrichtung verringert. Die Spitze ist vorzugsweise kegelförmig. Damit weist der zweite expandierbare Abschnitt des dritten Tubus im expandierten Zustand eine sich in proximaler Richtung verjüngende Ausdehnung quer zu seiner Längsachse auf. Dabei kann vorgesehen sein, dass der zweite expandierbare Abschnitt im expandierten Zustand seine maximale Ausdehnung in einem Bereich aufweist, der an den ersten Tubus angrenzt. Der Außendurchmesser des dritten Tubus ist geringer als der Innendurchmesser des zweiten Tubus. Das gilt auch für den zweiten expandierbaren Abschnitt im nicht expandierten Zustand. Im expandierten Zustand weist der zweite expandierbare Abschnitt jedoch einen Bereich mit einer maximalen Ausdehnung auf, die größer als der Außendurchmesser des zweiten Tubus ist und vorzugsweise dem Außendurchmesser des ersten Tubus an dessen offenem proximalem Ende entspricht.

Der dritte Tubus kann in den zweiten Tubus eingeführt werden und aus diesem entnommen werden, wenn sich der zweite expandierbare Abschnitt im nicht-expandierten Zustand befindet. Nach dem Führen des dritten Tubus durch den zweiten Tubus und das proximale offene Ende des ersten Tubus kann der zweite expandierbare Abschnitt mittels eines Fluids expandiert werden. Bildet der zweite expandierbare Abschnitt im expandierten Zustand eine Spitze, so liegt diese Spitze mit ihrer maximalen Ausdehnung - im Falle einer kegelförmigen Spitze mit ihrem maximalen Durchmesser - an dem offen proximalen Ende des ersten Tubus an. Die Spitze kann abgerundet sein. Die erfindungsgemäße Vorrichtung weist in dieser Ausführungsform dann eine Spitze auf, die das Einführen der erfindungsgemäßen Vorrichtung in das Hohlorgan und deren Führen an den Zielort innerhalb des Hohlorganes erleichtert.

Befindet sich die erfindungsgemäße Vorrichtung am Zielort, so liegt die erfindungsgemäße Vorrichtung mit ihrer Außenseite - das ist die Außenseite des ersten Tubus - an der Innenseite des Hohlorganes an. Dabei ist der zweite expandierbare Abschnitt im expandierten Zustand, während der erste expandierbare Abschnitt im nicht-expandierten Zustand ist. Wird nun der erste Tubus entfernt, beispielsweise indem er aus dem Hohlorgan herausgezogen wird, verbleibt der zweite Tubus am Zielort. Dabei ist die beschichtete Gewebeersatz-Trägeroberfläche nun der Innenseite des Hohlorganes zugewandt. Wird nun der erste expandierbare Abschnitt durch Zufuhr eines Fluids vom nicht-expandierten Zustand in den expandierten Zustand überführt, so gelangt der Gewebeersatz, der sich auf der Gewebeersatz-Trägeroberfläche befindet, in Kontakt mit der Innenseite des Hohlorgans. Vorzugsweise wird dabei der Gewebeersatz leicht an die Innenseite des Hohlorgans angedrückt.
Vor und nach dem Expandieren des ersten expandierbaren Abschnittes kann der dritte Tubus aus der erfindungsgemäßen Vorrichtung entnommen werden. Dazu wird der zweite expandierbare Abschnitt vom expandierten Zustand in den nicht-expandierten Zustand überführt. Anschließend kann der zweite expandierbare Abschnitt durch den zweiten Tubus geführt werden und damit der dritte Tubus aus dem zweiten Tubus hinausgeführt werden. Vorzugsweise wird der dritte Tubus aus dem zweiten Tubus entnommen, bevor der erste expandierbare Abschnitt in den expandierten Zustand überführt wird.

Es kann zweckmäßig sein, wenn der Gewebeersatz für einen vorgegebenen Zeitraum durch den zweiten Tubus in Kontakt mit der Innenseite des Hohlorgans gehalten wird. Dabei sollte sich der erste expandierbare Abschnitt im expandierten Zustand befinden, so dass der Gewebeersatz an der Innenseite des Hohlorganes anliegt. Die Länge des vorgegebenen Zeitraums kann von der Art des Hohlorgans, der Art des Gewebeersatzes, der Art der medizinischen Behandlung sowie den individuellen Gegebenheiten des Patienten abhängen. Bei einer Harnröhrenschlitzung sollte der Zeitraum 3 Tage oder mehr, bevorzugt 5 Tage oder mehr betragen. Nach Ablauf des Zeitraums sollte der Gewebeersatz mit dem Hohlorgan verwachsen sein. Die Gewebeersatz-Trägeroberfläche wird dann von dem Gewebeersatz gelöst und der erste expandierbare Abschnitt vom expandierten Zustand in den nicht-expandierten Zustand überführt. Anschließend kann der zweite Tubus aus dem Hohlorgan entfernt werden.

Es kann vorgesehen sein, dass die Längsachsen des ersten, zweiten und dritten Tubus koaxial zueinander verlaufen, wenn der zweite Tubus in den ersten Tubus und dritte Tubus in den zweiten Tubus eingeführt ist. Der Außendurchmesser dieser Variante der erfindungsgemäßen Vorrichtung hängt vom Durchmesser des Lumens ab, den das Hohlorgan, in das die Vorrichtung eingeführt werden soll, besitzt. Dem Fachmann ist bekannt, welcher Durchmesser zu wählen ist, um eine Vorrichtung zu erhalten, die in ein bestimmtes Hohlorgan eingeführt werden kann. Ist beispielsweise die Einführung in die Harnröhre vorgesehen, so kann der Außendurchmesser des ersten Tubus beispielsweise 10 bis 28 French betragen. Der Außendurchmesser des zweiten Tubus kann beispielsweise 4 bis 12 French betragen. Ein French entspricht 1/3 mm.

Der zweite Tubus soll an seinem proximalen Ende eine Öffnung zur Durchführung des dritten Tubus aufweisen. Der zweite Tubus kann einen Hohlmantel aufweisen, dessen Innenwand die Innenseite des zweiten Tubus und dessen Außenwand die Außenseite des zweiten Tubus bildet und der an seiner Stirnseite am proximalen Ende des zweiten Tubus geschlossen ist. An der Außenwand des zweiten Tubus ist der erste expandierbare Abschnitt nach Art des Ballons eines Ballonkatheters ausgebildet. Beim Einführen eines Fluids in den Hohlmantel des zweiten Tubus kann Druck auf den ersten expandierbaren Abschnitt ausgeübt werden, wodurch dieser in den expandierten Zustand gelangt. Der dritte Tubus soll an seinem proximalen Ende geschlossen sein, so dass beim Einführen eines Fluids in den dritten Tubus Druck auf den zweiten expandierbaren Abschnitt ausgeübt werden kann, wodurch dieser in den expandierten Zustand gelangt. Der erste Tubus ist erfindungsgemäß an seinem proximalen Ende offen. Der dritte Tubus wird vorzugsweise von dem zweiten expandierbaren Abschnitt des dritten Tubus verschlossen, wenn sich der zweite expandierbare Abschnitt im expandierten Zustand befindet.

Der erste Tubus kann ein erster Katheter sein, der zweite Tubus und der dritte Tubus können Ballonkatheter sein. Ist der zweite Tubus ein Ballonkatheter, so wird der erste expandierbare Abschnitt von dem Ballon des Ballonkatheters gebildet. Ist der dritte Tubus ein Ballonkatheter, so wird der zweite expandierbare Abschnitt von dem Ballon des Ballonkatheters gebildet.

Am proximalen Ende der Vorrichtung, also dem zweiten expandierbaren Abschnitt des dritten Tubus, können weitere Bestandteile der Vorrichtung angeordnet sein, beispielsweise eine Lichtquelle, eine optische Einrichtung, eine photographische Einrichtung wie eine Kamera oder Kombinationen davon. Die erfindungsgemäße Vorrichtung kann ferner Mittel zur Zufuhr und Abfuhr des oder der Fluide, die zum Expandieren des ersten expandierbaren Abschnitts, des zweiten expandierbaren Abschnitts oder von beiden erforderlich sind, benötigt werden, umfassen.

Am proximalen Ende sind der erste, zweite und dritte Tubus vorzugsweise offen. An das proximale Ende des dritten Tubus kann ein Schlauch zum Führen des Fluids in und aus dem dritten Tubus angeschlossen werden. An das proximale Ende des zweiten Tubus kann ebenfalls ein Schlauch zum Führen des Fluids in und aus den zweiten Tubus, d. h., wenn vorhanden, in dessen Hohlmantel, angeschlossen werden.

Der erste, zweite und dritte Tubus können aus Materialien bestehen, die nach dem Stand der Technik für Stents, Katheter und Ballonkatheter verwendet werden. Bei dem Material kann es sich beispielsweise um einen Kunststoff wie Silikon handeln. Die expandierbaren Abschnitte sollten aus einem elastischen Material bestehen, beispielweise Silikon. Bei den Fluiden kann es sich um eine Flüssigkeit handeln.

Weist die erfindungsgemäße Vorrichtung Befestigungsmittel auf, so können es die Befestigungsmittel insbesondere dann ermöglichen, den Gewebeersatz von der Gewebeersatz-Trägeroberfläche zu trennen, wenn der erste expandierbare Abschnitt vom expandierten Zustand in den nicht-expandierten Zustand überführt wird. Die Größe der Gewebeersatz-Trägeroberfläche hängt von der Größe des Gewebeersatzes ab, der an der Gewebeersatz-Trägeroberfläche befestigt werden soll. Wie bereits oben angegeben hat die Gewebeersatz-Trägeroberfläche vorzugsweise eine Länge von 3 bis 12 cm, stärker bevorzugt von 5 bis 10 cm, am stärksten bevorzugt von 8 cm. Die Länge der Gewebeersatz-Trägeroberfläche bezieht sich in dieser Variante der erfindungsgemäßen Vorrichtung auf deren Ausdehnung parallel zur Längsachse des zweiten Tubus. Die Ausdehnung des ersten expandierbaren Abschnittes in dieser Richtung kann gleich oder größer als die Länge der Gewebeersatz-Trägeroberfläche sein. In Umfangsrichtung kann die Ausdehnung der Gewebeersatz-Trägeroberfläche dem Umfang des zweiten Tubus oder einem Teil des Umfangs des zweiten Tubus entsprechen. Der Umfang des zweiten Tubus sollte dem Umfang des ersten expandierbaren Abschnittes im nicht-expandierten Zustand entsprechen.

Nach Maßgabe der Erfindung ist ferner ein Verfahren zur Herstellung einer erfindungsgemäßen Vorrichtung vorgesehen, das die Schritte umfasst:
(a) Aufbringen des Gewebeersatzes auf die Oberfläche zum Tragen des Gewebeersatzes (Gewebeersatz-Trägeroberfläche), die an dem ersten Abschnitt des zweiten Tubus ausgebildet ist;
(b) Einführen des zweiten Tubus in den ersten Tubus; und
(c) Einführen des dritten Tubus in den zweiten Tubus.

Das Verfahren kann ferner den Schritt umfassen:
(d) Expandieren des zweiten Abschnittes des dritten Tubus.

In Schritt (a) kann der Gewebeersatz vorzugsweise mittels Befestigungselementen lösbar an der Gewebeersatz-Trägeroberfläche befestigt werden. Weitere Einzelheiten zu dem erfindungsgemäßen Verfahren sind vorstehend bereits im Zusammenhang mit der erfindungsgemäßen Vorrichtung beschrieben worden.

In einer Ausführungsform ist die erfindungsgemäße Vorrichtung eine Anordnung zum Einbringen eines Gewebeersatzes in ein Hohlorgan, umfassend
- als ersten Tubus einen ersten, als Schutzelement dienenden Katheter mit einem offenen proximalen Ende; und
- als zweiten Tubus einen zweiten, als Träger dienenden Katheter zur Einführung in den ersten Katheter, wobei der zweite Katheter einen durch Zufuhr eines Fluids ersten expandierbaren Abschnitt mit einer Oberfläche zum Tragen des Gewebeersatzes aufweist.

Zusätzlich weist die Anordnung als dritten Tubus einen dritten Katheter zur Führung durch den zweiten Katheter und das offene proximale Ende des ersten Katheters umfassen. Vorzugsweise weist der dritte Katheter an seinem proximalen Ende einen durch Zufuhr eines Fluids zweiten expandierbaren Abschnitt auf.

Die erfindungsgemäße Anordnung kann den Gewebeersatz umfassen. Sie kann ferner Befestigungsmittel zur lösbaren Befestigung des Gewebeersatzes an der Gewebeersatz-Trägeroberfläche umfassen. Einzelheiten der Anordnung sind vorstehend im Zusammenhang mit der erfindungsgemäßen Vorrichtung beschrieben worden. Dabei entspricht der erste Tubus dem ersten Katheter, der zweite Tubus dem zweiten Katheter und der dritte Tubus dem dritten Katheter. Der zweite und dritte Tubus können Ballonkatheter sein.

### Membran

Unter einer Membran wird hier insbesondere ein flächiges poröses Gebilde verstanden. Die Membran(en) sollten biologischen und nicht synthetischen Ursprungs sein. Damit sind die Membranen vollständig abbaubar, was eine Kalzifizierung oder Abstoßung der Membranen in der Zeit nach der Implantation verhindert. Die Membran kann Zellen oder Zellbestandteile beinhalten, was aber nicht erforderlich ist. Die Anwesenheit von zum Beispiel vitalen Mukosagewebezellen auf der Membran erlaubt die Generierung von neuem Gewebe durch körpereigene Zellen. Hiermit erübrigt sich die Notwendigkeit der dauerhaften Haltbarkeit des Gewebeersatzes. Alternativ kann die Membran oberflächenbehandelt sein. Beispielsweise kann die Membran mit Wachstumsfaktoren beschichtet oder strukturell so verändert sein, dass nach der Implantation des Gewebeersatzes die umgebenden Zellen aus dem Hohlorgan zu der Membran gezogen und sich auf dieser vermehren und somit eine Regeneration des Hohlorganes induzieren. Die Membran ist vorzugsweise eine biologisch kompatible Membran, z. B. eine Membran aus Kollagen.

### Membranverbund

Vorzugsweise ist die Membran Bestandteil eines Membranverbundes, der zumindest eine erste Membran und zumindest eine zweite umfasst, wobei die erste Membran und die zweite Membran an ihren Flachseiten aneinander angrenzen. Beide Membranen sind vorzugsweise biologisch kompatible Membranen, z. B. Membranen aus Kollagen.

Ein Membranverbund aus den beiden Membranen lässt sich vorteilhaft durch Verpressen der ersten und zweiten Membran und/oder durch Vernetzen, insbesondere durch Photovernetzen, der ersten und zweiten Membran herstellen. Ein so erhaltener Membranverbund hat eine ausreichende mechanische Stabilität, so dass er für die Herstellung der erfindungsgemäßen Vorrichtung verwendet werden kann, die insbesondere zur Rekonstruktion eines Hohlorgans eingesetzt werden kann.

Zur Herstellung eines Membranverbundes kann wie folgt vorgegangen werden:
(a1) Bereitstellen von zwei ersten Membranen und Überführen der ersten Membranen in einen gequollenen Zustand;
(a2) Bereitstellen einer zweiten Membran und Lyophilisieren der zweiten Membran;
(a3) Anordnen der zweiten Membran zwischen den beiden ersten Membranen; und
(a4) Herstellen eines Verbundes aus den ersten Membranen und der zweiten Membran.

Bevorzugt wird der Membranverbund durch Verpressen und/oder Vernetzen der ersten und der zweiten Membran hergestellt. Das Verpressen erfolgt vorzugsweise unter einem Druck von 5 bis 5000 kN/cm², stärker bevorzugt 10 bis 1000 kN/cm² und noch stärker bevorzugt bei 50 bis 150 kN/cm² und am stärksten bevorzugt bei 100 kN/cm². Bei Drücken, die geringer als 5 kN/cm² sind, kann möglicherweise kein hinreichend fester Verbund der Membranen erreicht werden, während bei Drücken über 5000 kN/cm² die Membranen, insbesondere deren Gerüst- und Porenstruktur, beschädigt werden können.

Es ist bevorzugt, das Verpressen bei einer Temperatur durchzuführen, die gegenüber der Umgebungstemperatur leicht erhöht ist, bevorzugt bei 25 bis 50 °C, stärker bevorzugt bei 35 bis 40 °C. Die leicht erhöhte Temperatur fördert die Beweglichkeit der Kollagenfasern, ohne deren dreidimensionale Struktur zu ändern. Das Verpressen wird vorzugsweise über einen Zeitraum von 1 min bis 2 h, stärker bevorzugt 0,5 bis 1,5 h und besonders bevorzugt 3 bis 13 Minuten durchgeführt.

Alternativ oder zusätzlich zum Verpressen kann der Membranverbund einer photochemischen Behandlung unterzogen werden, um eine Vernetzung der Kollagenfasern der ersten und zweiten Membran zu erreichen. Die Photopolymerisation erhöht die Steifigkeit des Membranverbundes. Die photochemische Behandlung kann beispielsweise unter Einwirkung von Ultraviolett-A-Strahlen (UVA) erfolgen. Es kann vorgesehen sein, dass die Membran vor Einwirkung der UVA-Strahlen mit einer lichtsensibilisierenden Substanz wie beispielsweise Riboflavin oder Vitamin B2 getränkt wird.

Die photochemische Vernetzung wird vorzugsweise mit sichtbarem Licht durchgeführt. Vorzugsweise hat das Licht eine Wellenlänge von 380 bis 600 nm, stärker bevorzugt von 425 bis 525 nm, besonders bevorzugt 475 nm. Die Vernetzung wird vorzugsweise über einen Zeitraum von 10 min bis 2 h, besonders bevorzugt 0,5 bis 1,5 h durchgeführt.

Die erste Membran ist in einer Ausführungsform der Erfindung equinen oder bovinen Ursprungs und die zweite Membran tierischen oder humanen Ursprungs, wobei die zweite Membran anderen Ursprungs als die erste Membran ist. Beispielsweise kann die zweite Membran porzinen Ursprungs sein.

Vorzugsweise weist der erfindungsgemäß vorgesehene Membranverbund zwei erste Membranen und eine zweite Membran auf, wobei die zweite Membran zwischen den beiden ersten Membranen angeordnet ist.

Weitere Einzelheiten zum Membranverbund und Verfahren zu seiner Herstellung können DE 10 2010 001 271 A1, Absätze [0014] bis [0032] und [0044 bis 0048] entnommen werden, deren Inhalt hierin durch Bezugnahme aufgenommen ist.

### Mukosagewebezellen

Die erfindungsgemäß vorgesehenen Mukosagewebezellen sind vorzugsweise autologe Mukosagewebezellen, besonders bevorzugt autologe orale Mukosagewebezellen. Bei Verwendung autologer Mukosagewebezellen ist der erfindungsgemäß vorgesehene Gewebeersatz insbesondere für die Reparatur und/oder den Ersatz von epithelialem Gewebe an einer Schlitzung eines Harnorgans geeignet.

Besonders geeignete autologe orale Mukosagewebezellen sind Mundschleimhautgewebezellen. Mundschleimhautgewebezellen verfügen über ein starkes Proliferationspotential und sind über relativ nicht-invasive Biopsien zugänglich, was das Mundschleimhautepithelgewebe zu einer attraktiven Zellquelle für autologe Therapien macht.

Der Begriff "autologe Zellen" bezieht sich dabei auf Zellen, die von dem Individuum stammen, für das das erfindungsgemäße Implantat bestimmt ist.

Vorzugsweise weist der Gewebeersatz eine oder mehrere Schichten von Mukosagewebezellen auf. Diese Schichten befinden sich vorzugsweise auf der der Gewebeersatz-Trägeroberfläche abgewandten Flächenseite der zumindest einen Membran oder, wenn ein Membranverbund vorgesehen ist, auf der der Gewebeersatz-Trägeroberfläche abgewandten, äußeren Flächenseite des Membranverbundes.

Weitere Einzelheiten zu Mukosagewebezellen können DE 10 2010 001 271 A1, Absätze [0033] und [0034] entnommen werden, deren Inhalt hierin durch Bezugnahme aufgenommen ist.

Ein Gegenstand der vorliegenden Erfindung ist es, eine Vorrichtung bereitzustellen, die das Einführen eines Gewebeersatzes (Grafts) in ein dünnes röhrenförmiges Organ ermöglicht, ohne dass der Gewebeersatz (Graft) verletzt oder beschädigt wird.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Darstellung einer ersten Ausführungsform der erfindungsgemäßen Vorrichtung, wobei der erste expandierbare Abschnitt und der zweite expandierbare Abschnitt jeweils im nicht-expandierten Zustand gezeigt sind;
- Fig. 2: eine schematische Darstellung der in Fig. 1 gezeigten ersten Ausführungsform der erfindungsgemäßen Vorrichtung, wobei der erste expandierbare Abschnitt im nicht-expandierten Zustand und der zweite expandierbare Abschnitt im expandierten Zustand gezeigt sind;
- Fig. 3: eine schematische Darstellung der in Fig. 1 gezeigten ersten Ausführungsform der erfindungsgemäßen Vorrichtung, wobei der erste expandierbare Abschnitt im nicht-expandierten Zustand und der zweite expandierbare Abschnitt im expandierten Zustand gezeigt sind, nach Einführung in die Harnröhre;
- Fig. 4: eine schematische Darstellung der in Fig. 1 gezeigten ersten Ausführungsform der erfindungsgemäßen Vorrichtung, wobei der erste expandierbare Abschnitt im nicht-expandierten Zustand und der zweite expandierbare Abschnitt im expandierten Zustand gezeigt sind, nach Einführung in die Harnröhre und Entfernung des ersten Tubus;
- Fig. 5: eine schematische Darstellung der in Fig. 1 gezeigten ersten Ausführungsform der erfindungsgemäßen Vorrichtung, wobei der erste expandierbare Abschnitt im nicht-expandierten Zustand gezeigt ist, nach Einführung in die Harnröhre und Entfernung des ersten Tubus und dritten Tubus;
- Fig. 6: eine schematische Darstellung der in Fig. 1 gezeigten ersten Ausführungsform der erfindungsgemäßen Vorrichtung, wobei der erste expandierbare Abschnitt im expandierten Zustand gezeigt ist, nach Einführung in die Harnröhre und Entfernung des ersten Tubus und dritten Tubus;
- Fig. 7: eine schematische Darstellung der in Fig. 1 gezeigten ersten Ausführungsform der erfindungsgemäßen Vorrichtung, wobei der erste expandierbare Abschnitt im expandierten Zustand gezeigt ist, nach Einführung in die Harnröhre und Entfernung des ersten Tubus und dritten Tubus;
- Fig. 8: eine schematische Darstellung der in Fig. 1 gezeigten ersten Ausführungsform der erfindungsgemäßen Vorrichtung, wobei der erste expandierbare Abschnitt im expandierten Zustand gezeigt ist, nach Einführung in die Harnröhre, Entfernung des ersten Tubus und dritten Tubus sowie Ablösung des Gewebeersatzes von der Gewebeersatz-Trägeroberfläche;
- Fig. 9: eine schematische perspektivische Darstellung einer nicht erfindungsgemäßen Vorrichtung;
- Fog. 10: eine schematische Schnittdarstellung der in Fig. 9 gezeigten nicht erfindungsgemäßen Vorrichtung;
- Fig. 11: eine schematische perspektivische Darstellung der in Fig. 9 gezeigten nicht erfindungsgemäßen Vorrichtung ohne Schutzelement; und
- Fig. 12: eine schematische Schnittdarstellung der in Fig. 9 gezeigten nicht erfindungsgemäßen Vorrichtung ohne Schutzelement.

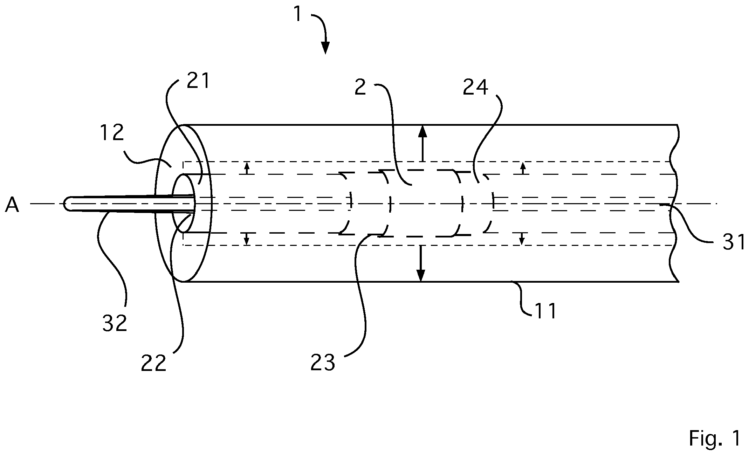

Die in den Figuren 1 bis 8 gezeigte erste Ausführungsform einer erfindungsgemäßen Vorrichtung 1 weist einen ersten Tubus 11, einen zweiten Tubus 21 und einen dritten Tubus 31 auf. Die Außenseite des ersten Tubus 11 bildet die Außenseite der erfindungsgemäßen Vorrichtung 1. An seinem proximalen Ende ist der erste Tubus 11 unter Ausbildung der ersten Öffnung 12 offen, um die Durchführung des dritten Tubus 31 zu ermöglichen.

In den ersten Tubus 11 ist der zweite Tubus 21 eingeführt, der sich bis zum offenen proximalen Ende des ersten Tubus 11 erstreckt, wobei die Längsachse des ersten Tubus 11 und die Längsachse des zweiten Tubus 21 koaxial auf der Längsachse A der Vorrichtung 1 liegen. Die Außenseite des zweiten Tubus 21 ist von der Innenseite des ersten Tubus 11 beabstandet. An seinem proximalen Ende weist der zweite Tubus 21 eine zweite Öffnung 22 zur Durchführung des dritten Tubus 31 auf. Die zweite Öffnung 22 des zweiten Tubus 21 und die erste Öffnung 12 des ersten Tubus 11 liegen konzentrisch zueinander.

Der zweite Tubus 21 ist ein Ballonkatheter mit einem expandierbaren Abschnitt, dem Ballon. Der Ballon bildet den ersten expandierbaren Abschnitt 23 der Vorrichtung 1 und ist vom proximalen Ende des zweiten Tubus 21 beabstandet ausgebildet. Der zweite Tubus 21 kann einen Hohlmantel aufweisen, der an seiner Stirnseite am proximalen Ende des zweiten Tubus geschlossen ist. Dabei umschließt die Innenseite des Hohlmantels die zweite Öffnung 22. In den Hohlmantel kann ein Fluid eingeführt werden, das die Überführung des ersten expandierbaren Abschnitts 23 vom nicht-expandierbaren Zustand - der in Fig. 1 gezeigt ist - in den expandierbaren Zustand und umgekehrt ermöglicht. Im nicht-expandierten Zustand des ersten expandierbaren Abschnitts 23 kann der zweite Tubus 21 in den ersten Tubus 11 eingeführt und aus diesem herausgeführt werden. Die Außenwand des Hohlmantels bildet die Außenwand des zweiten Tubus 21. Der zweite expandierbare Abschnitt 23 ist an der Außenwand des Hohlmantels ausgebildet. Die Außenfläche des ersten expandierbaren Abschnittes 23, d. h. die Fläche, die der Innenseite des ersten Tubus 11 zugewandt ist, bildet die Gewebeersatz-Trägeroberfläche 24. Die Gewebeersatz-Trägeroberfläche 24 ist angeraut und noppenförmig, so dass der Gewebeersatz darauf fixiert und gleichzeitig die Durchblutung des Wundbettes zwischen den Druckstellen maximal gewährleistet werden kann. An der Gewebeersatz-Trägeroberfläche 24 kann der Gewebeersatz 2 lösbar angebracht sein, beispielsweise mittels Befestigungsmitteln (nicht gezeigt). In Fig. 1 ist ein Gewebeersatz 2 an der Gewebeersatz-Trägeroberfläche 24 angebracht. Dabei handelt es sich um einen mit Mukosagewebezellen besiedelten Membranverbund, der unter dem Handelsnamen "MukoCell" kommerziell erhältlich ist. Anstatt MukoCell kann auch ein anderer Gewebeersatz, beispielsweise natives Gewebe wie ein Mundschleimhaut-Segment oder eine Membran ohne Zellbestanteile, an der Gewebeersatz-Trägeroberfläche 24 fixiert werden. Einzelheiten des MukoCell-Membranverbundes sind oben im Abschnitt "Membranverbund" beschrieben worden. Es ist in Fig. 1 zu erkennen, dass die äußere Oberfläche des Gewebeersatzes 2 von der Innenseite des ersten Tubus 11 beabstandet ist.

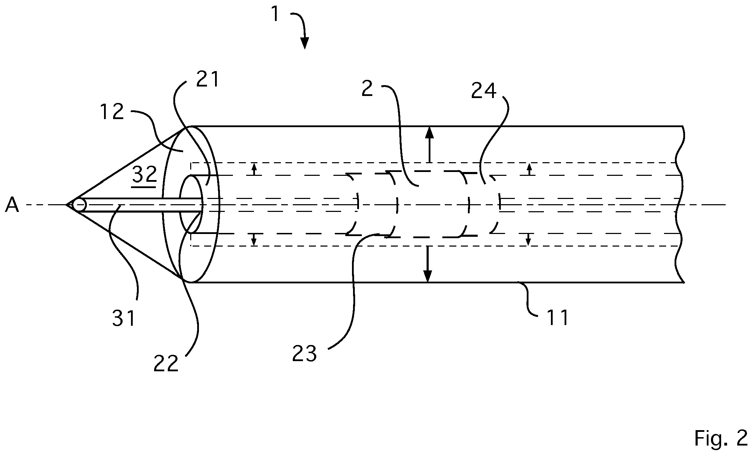

Durch den zweiten Tubus 21 ist der dritte Tubus 31 geführt. Er tritt durch die zweite Öffnung 22 am proximalen Ende des zweiten Tubus 21 und durch die erste Öffnung 12 am proximale Ende des ersten Tubus 11 hindurch. Er kann am proximalen Ende des ersten und zweiten Tubus 11, 21 über deren Stirnseiten überstehen. Der dritte Tubus kann an seinem proximalen Ende geschlossen sein. Er kann in seinem überstehenden Bereich einen expandierbaren Abschnitt aufweisen, der der zweite expandierbare Abschnitt 32 der erfindungsgemäßen Vorrichtung 1 ist. Durch den dritten Tubus 31 kann ein Fluid geführt werden, um den zweiten expandierbaren Abschnitt 32 vom nicht-expandierten Zustand - der in Fig. 1 gezeigt ist - in den expandierten Zustand - der in Fig. 2 gezeigt ist - zu überführen. Im nicht-expandierten Zustand des zweiten expandierbaren Abschnittes 32 kann der dritte Tubus 31 in den zweiten Tubus 21 eingeführt und aus diesem herausgeführt werden.

Es ist in Fig. 2 zu erkennen, dass der zweite expandierbare Abschnitt 32 im expandierten Zustand eine abgerundete kegelförmige Spitze bildet, um das gleitende und schonende Einführen der erfindungsgemäßen Vorrichtung, insbesondere des ersten Tubus 11 und somit auch des zweiten Tubus 21, der den Gewebeersatz trägt, in das Hohlorgan zu gewährleisten. Dabei grenzt der zweite expandierbare Abschnitt 32 mit dem Bereich seiner größten Ausdehnung an das proximale Ende des ersten Tubus 11 an, so dass er die erste Öffnung 12 des ersten Tubus 11 verschließt. Dabei verschließt er auch die zweite Öffnung 22 des zweiten Tubus 21.

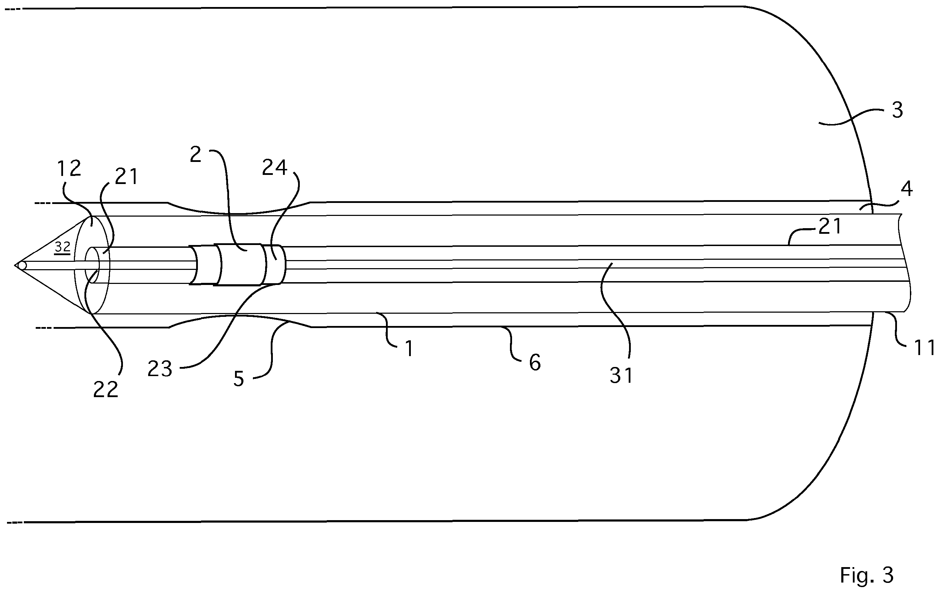

In dem in Fig. 2 gezeigten Zustand, d. h. der erste expandierbare Abschnitt 23 ist im nicht-expandierten Zustand und der zweite expandierbare Abschnitt 32 ist im expandierten Zustand, kann die erfindungsgemäße Vorrichtung, wie in Fig. 3 gezeigt ist, in das Lumen eines Hohlorgans, beispielsweise die im Penis 3 ausgebildete Harnröhre 4, eingeführt werden. Dabei wird die erfindungsgemäße Vorrichtung 1 mit ihrem proximalen Ende in die Harnröhre 4 eingeführt, wobei die erfindungsgemäße Vorrichtung 1 mit der Außenseite des ersten Tubus an der Wandung 6 der Harnröhre 4 anliegt. Aufgrund der abgerundeten kegelförmigen Spitze der Vorrichtung 1, die von dem zweiten expandierbaren Abschnitt 32, der sich im expandierten Zustand befindet, gebildet wird, ist dies leicht möglich. Die Einführung der erfindungsgemäßen Vorrichtung kann mittels Techniken und Hilfsmitteln vorgenommen werden, die aus dem Stand der Technik bei Kathetern bekannt sind. Der erste Tubus 11 hat die Aufgabe, den auf die Gewebeersatz-Trägeroberfläche 24 des zweiten Tubus 21 aufgebrachten Gewebeersatz beim Einführen in das Hohlorgan vor Reibungen und weiteren potentiellen mechanischen Schädigungen als Mantel zu schützen. Die erfindungsgemäße Vorrichtung 1 wird soweit in die Harnröhre 4 eingeführt werden, bis sie den Zielort 5 (beispielsweise die Striktur oder Verengung) erreicht hat. Der Zielort 5 ist der Ort, an dem der Gewebeersatz 2 an die Innenseite des Hohlorgans aufgebracht werden soll, und zwar zweckmäßigerweise so, dass der Gewebeersatz 2 die vorher geschlitzte Verengung vollständig abdeckt. Im Falle einer Harnröhre 4 kann das der Ort einer Schlitzung sein, die zur Behandlung einer Harnröhrenverengung vorgenommen worden ist.

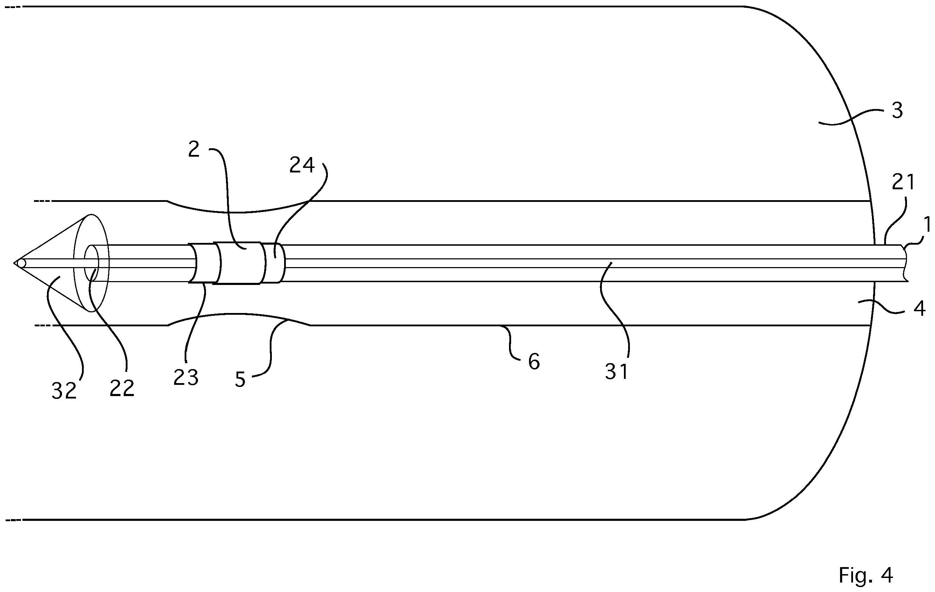

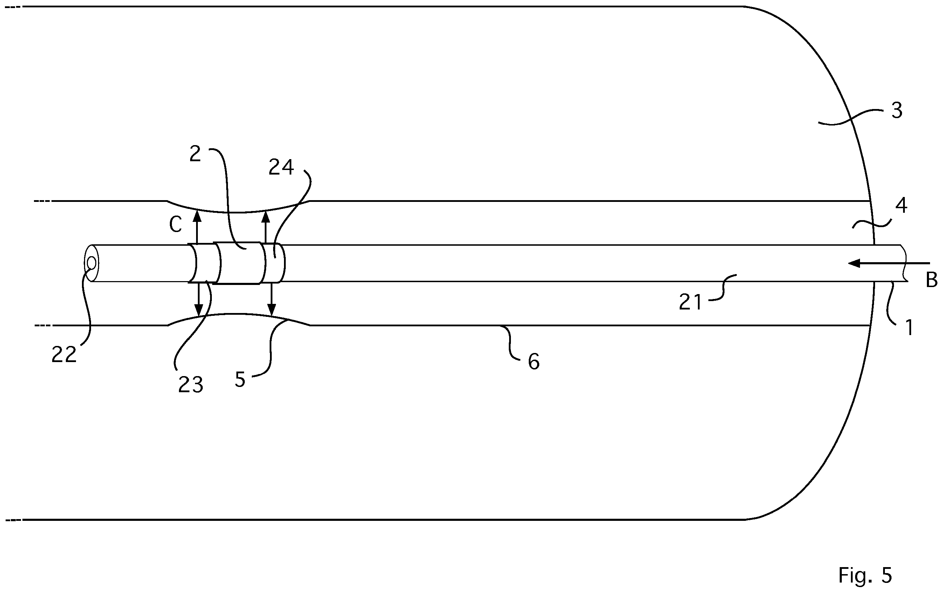

Die erfindungsgemäße Vorrichtung 1 hat den Zielort 5 erreicht, wenn der erste expandierbare Abschnitt 23 dem Zielort 5 gegenüberliegt. Anschließend wird nun der erste Tubus 11 entfernt, indem er aus der Harnröhre 4 herausgezogen wird. Der zweite Tubus 21 mit dem Gewebeersatz 2 und der dritte Tubus 31 verbleiben in der Harnröhre 4, ohne ihre Lage in Bezug auf den Zielort 5 zu verändern (Fig. 4). Nachdem der erste Tubus 11 aus der Harnröhre 4 entfernt worden ist, kann der zweite expandierbare Abschnitt 32 des dritten Tubus 31 vom expandierten Zustand in den nicht-expandierten Zustand überführt werden. Anschließend kann der dritte Tubus 31, einschließlich des zweiten expandierbaren Abschnittes 32, aus der Harnröhre 4 durch den zweiten Tubus 21 herausgezogen werden. Damit verbleibt nur der zweite Tubus 21 mit dem Gewebeersatz 2 in der Harnröhre 4, und zwar ohne seine Lage in Bezug auf den Zielort 5 zu verändern (Fig. 5).

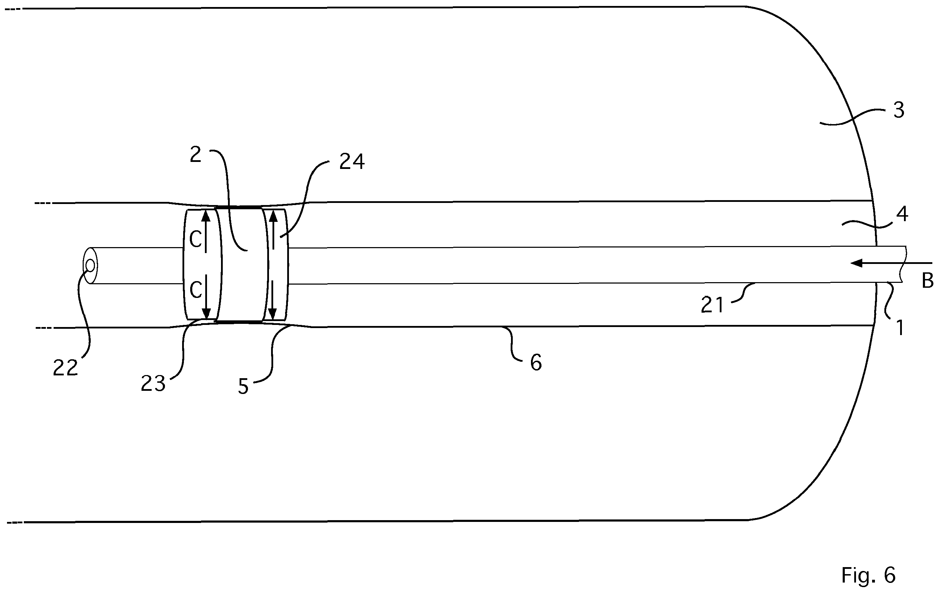

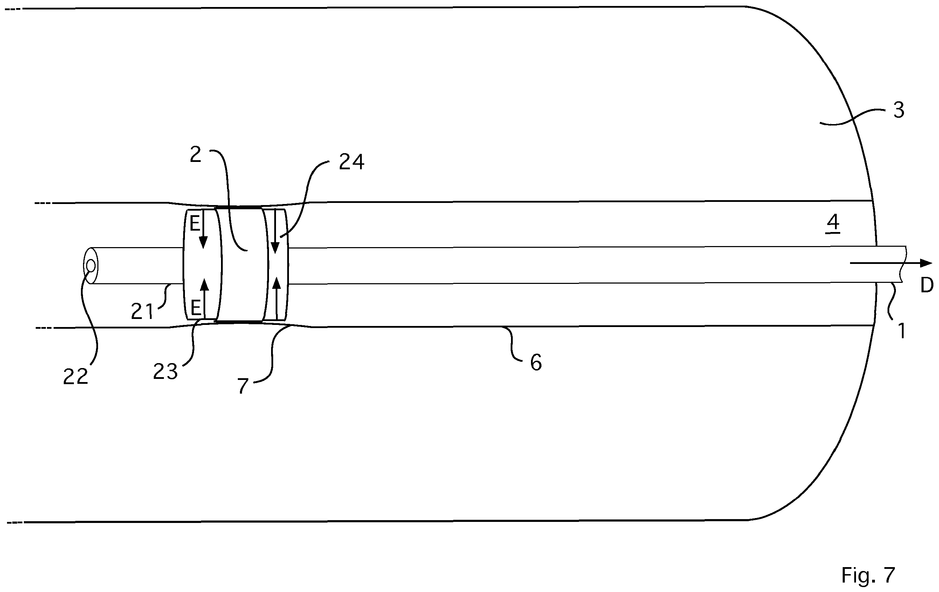

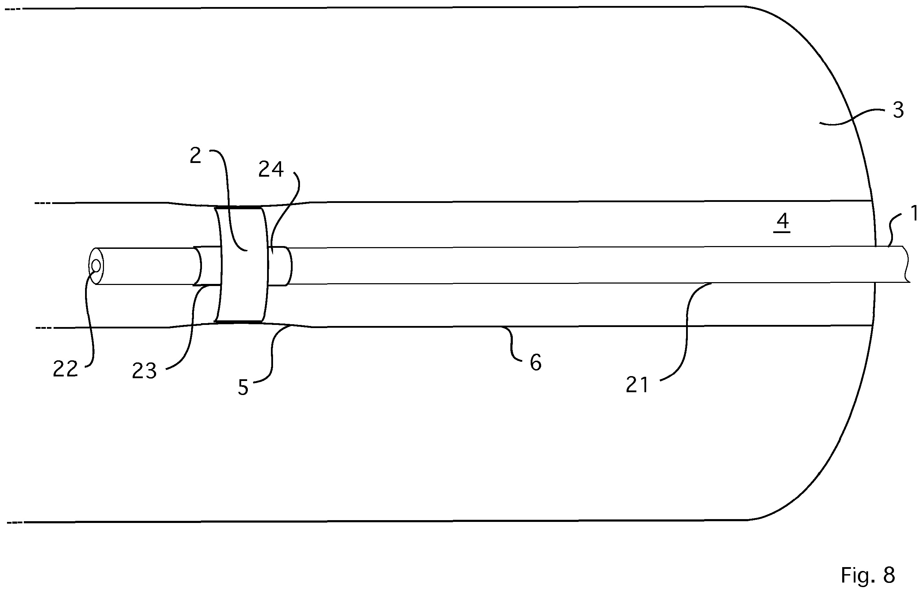

Durch Zufuhr eines Fluids (Pfeil B in Fig. 5) in den Hohlmantel des zweiten Tubus 21 kann nun der erste expandierbare Abschnitt 23 in den expandierten Zustand (Pfeile C) überführt werden. Dabei gelangt der Gewebeersatz 2 in Kontakt mit der Wandung 6 der Harnröhre 4 am Zielort 5, d. h. der Verengung oder Striktur (Fig. 6). Der Gewebeersatz 2 wird nun für eine vorgegebene Zeitdauer, beispielsweise 5 bis 25 Tage, in Kontakt mit der Wandung gehalten, wobei er mittels des ersten expandierbaren Abschnittes 23 leicht an die Wandung 6 der Harnröhre angedrückt wird. Innerhalb dieser Zeit wächst er an der Wandung 6 an und verschließt dabei die Schlitzung. Nach Ablauf der vorgegebenen Zeitdauer wird der Gewebeersatz 2 von der Gewebeersatz-Trägeroberfläche 24 des ersten expandierbaren Abschnitt 23 des zweiten Tubus 21 gelöst. Durch Abführen des Fluids (Pfeil D in Fig. 7) aus dem Hohlmantel des zweiten Tubus 21 wird der erste expandierbare Abschnittes 23 vom expandierten Zustand in den nicht-expandierten Zustand überführt (Pfeile E), wobei der Gewebeersatz 2 an der Harnröhre 4 verbleibt (Fig. 8). Anschließend kann der zweite Tubus 21 mit seinem ersten expandierbaren Abschnitt 23 aus der Harnröhre 4 herausgezogen werden.

Bei den drei Tubussen 11, 21, 31 kann es sich um Katheter handeln. Die drei Tubusse 11, 21, 31 bilden eine Ausführungsform der erfindungsgemäßen Anordnung.

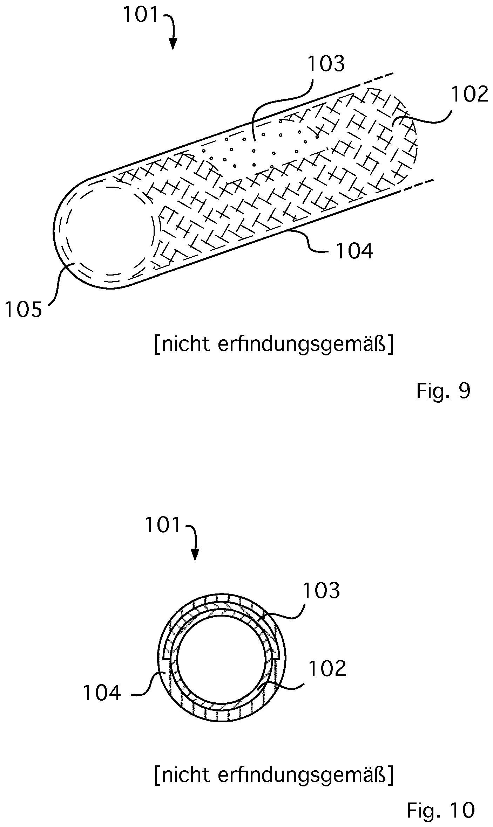

Die in den Figuren 9 bis 12 gezeigte nicht erfindungsgemäße Vorrichtung 101 weist einen Stent 102 als Träger auf. Der Stent ist ein rohrförmiges Gebilde, das an seinen Stirnseiten offen ist. Ein Teil der Außenseite des Stents 102 bildet die Gewebeersatz-Trägeroberfläche, auf die der Gewebeersatz 103 aufgebracht ist. Der Stent 102 und der Gewebeersatz 103 werden durch eine Schutzhülle 104 geschützt, die eine Stirnseite 105 der Stents 102 und zumindest den Teil der Außenseite des Stents 102 umgibt, der die Gewebeersatz-Trägeroberfläche bildet. Es ist insbesondere in Fig. 10 zu erkennen, dass der Gewebeersatz 103 zwischen der Innenseite der Schutzhülle 104 und der Außenseite des Stents 102 liegt.

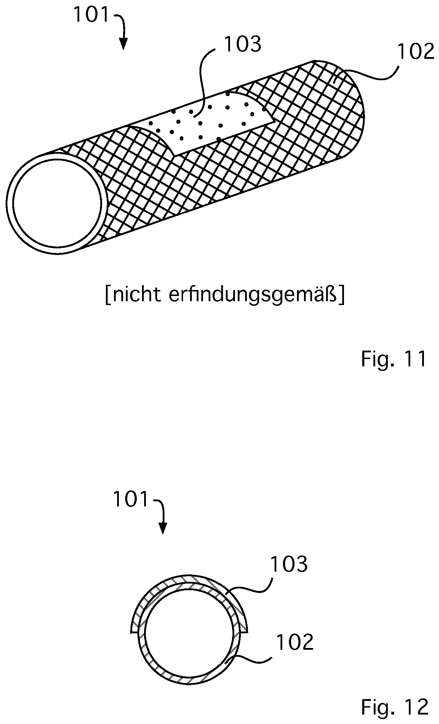

Zur Herstellung der zweiten Ausführungsform wird ein Stent 102 bereitgestellt und auf die Gewebeersatz-Trägeroberfläche des Stents 102 der Gewebeersatz 103 aufgebracht (siehe Figuren 11 und 12). Anschließend wird die Schutzhülle 104 über die erste Stirnseite 105 des Stents geführt, so dass der Gewebeersatz 103 zwischen der Innenseite der Schutzhülle 104 und der Außenseite des Stents 102 liegt (siehe Figuren 9 und 10). Die so erhaltene zweite Ausführungsform der erfindungsgemäßen Vorrichtung 101 kann damit, mit ihrer Stirnseite 105 voran, in den Lumen des Hohlorgans eingeführt werden, und zwar bis zum Zielort. Sodann kann die Schutzhülle 105, beispielsweise mittels eines Messers, das durch den Hohlraum des Stents 102 geführt wird, zerschnitten und dann entfernt werden. Der Gewebeersatz liegt dann frei und kann in Kontakt mit der Innenseite des Hohlorgans gelangen. Sobald der Gewebeersatz mit dem Hohlorgan verwachsen ist, kann - falls das gewünscht ist - der Stent aus dem Hohlorgan entfernt werden, beispielsweise indem er herausgezogen wird.

### Bezugszeichenliste

- 1: Vorrichtung
- 2: Gewebeersatz
- 3: Penis
- 4: Harnröhre
- 5: Zielort
- 6: Wandung

- 11: erster Tubus
- 12: erste Öffnung

- 21: zweiter Tubus
- 22: zweite Öffnung
- 23: erster expandierbarer Abschnitt
- 24: Gewebeersatz-Trägeroberfläche

- 31: dritter Tubus
- 32: zweiter expandierbarer Abschnitt

- 101: Vorrichtung
- 102: Stent
- 103: Gewebeersatz
- 104: Schutzhülle
- 105: erste Stirnseite des Stents

## Patentansprüche

1. Vorrichtung zum Einbringen eines Gewebeersatzes (2, 103) in ein Hohlorgan (4), umfassend einen Träger (21, 102) mit einer Oberfläche zum Tragen des Gewebeersatzes (2, 103) und ein Schutzelement (11, 104) zum Schutz des Gewebeersatzes (2, 103) während des Einbringens der Vorrichtung (1, 101) in das Hohlorgan (4), **dadurch gekennzeichnet, dass** die Vorrichtung umfasst:
- einen als Schutzelement dienenden ersten Tubus (11) mit einem offenen proximalen Ende (12);
- einen als Träger dienenden zweiten Tubus (21), der in den ersten Tubus (11) einführbar ist und einen durch Zufuhr eines Fluids ersten expandierbaren Abschnitt (23) mit der Oberfläche (24) zum Tragen des Gewebeersatzes (2) aufweist; und
- einen dritten Tubus (31), der durch den zweiten Tubus (21) und das offene proximale Ende (12) des ersten Tubus (11) führbar ist, wobei der dritte Tubus (31) an seinem proximalen Ende einen durch Zufuhr eines Fluids zweiten expandierbaren Abschnitt (32) aufweist.

2. Vorrichtung nach Anspruch 1, **dadurch gekennzeichnet, dass** der Gewebeersatz (2, 103) ein Implantat ist.

3. Vorrichtung nach einem der vorstehenden Ansprüche, **dadurch gekennzeichnet, dass** der Gewebeersatz (2, 103) aus der Gruppe ausgewählt ist, die Haut; Mundschleimhaut; natives Gewebe, künstlich hergestelltes Gewebe; eine Membran; eine Membran, die Zellen und/oder Zellbestandteile aufweist; eine Membran, die keine Zellen und Zellbestandteile aufweist, eine Membran mit behandelter Oberfläche sowie einen Membranverbund umfasst.

4. Vorrichtung nach Anspruch 3, **dadurch gekennzeichnet, dass** die Membran oder der Membranverbund mit Mukosagewebezellen besiedelt ist.

5. Vorrichtung nach einem der vorstehenden Ansprüche, **dadurch gekennzeichnet, dass** die Längsachsen des ersten, zweiten und dritten Tubus (11, 21, 31) koaxial zueinander verlaufen.

6. Vorrichtung nach einem der vorstehenden Ansprüche, **dadurch gekennzeichnet, dass** sie ferner Befestigungsmittel zur lösbaren Befestigung des Gewebeersatzes (2) an der Oberfläche (24) des ersten expandierbaren Abschnittes (23) aufweist.

7. Vorrichtung nach einem der vorstehenden Ansprüche, **dadurch gekennzeichnet, dass** der zweite expandierbare Abschnitt (32) des dritten Tubus (31) eine maximale Ausdehnung quer zur Längsachse (A) des dritten Tubus (31) aufweist, die der Ausdehnung des ersten Tubus (11), quer zu dessen Längsachse (A), entspricht oder dessen Ausdehnung übersteigt.

8. Vorrichtung nach einem der vorstehenden Ansprüche, **dadurch gekennzeichnet, dass** der zweite expandierbare Abschnitt (32) des dritten Tubus (31) im expandierten Zustand eine sich in proximaler Richtung verjüngende Ausdehnung quer zu dessen Längsachse (A) aufweist.

9. Verfahren zur Herstellung einer Vorrichtung gemäß einem der Ansprüche 1 bis 8, **dadurch gekennzeichnet, dass** es die Schritte umfasst:
(a) Aufbringen des Gewebeersatzes (2) auf die Oberfläche (24) zur Aufnahme des Gewebeersatzes (2), die an dem ersten expandierbaren Abschnitt (23) des zweiten Tubus (21) ausgebildet ist;
(b) Einführen des zweiten Tubus (21) in den ersten Tubus (11); und
(c) Führen des dritten Tubus durch den zweiten Tubus und das offene proximale Ende (12) des ersten Tubus (11).

10. Vorrichtung nach einem der Ansprüche 1 bis 8, **dadurch gekennzeichnet, dass** die Vorrichtung eine Anordnung ist, die einen Träger (21, 102) mit einer Oberfläche zum Tragen des Gewebeersatzes (2, 103) und ein Schutzelement (11, 104) zum Schutz des Gewebeersatzes während des Einbringens der Anordnung in das Hohlorgan (4 umfasst, wobei:
- der als Schutzelement dienende erste Tubus ein erster Katheter (11) mit einem offenen proximalen Ende (12) ist;
- der als Träger dienende zweite Tubus ein zweiter Katheter (21) zur Einführung in den ersten Katheter (11) ist, wobei der zweite Katheter (21) einen durch Zufuhr eines Fluids ersten expandierbaren Abschnitt (23) mit einer Oberfläche (24) zur Aufnahme des Gewebeersatzes (2) aufweist; und
- der dritte Tubus ein dritter Katheter (31) zum Führen durch den zweiten Katheter (21) und das offene proximale Ende (12) des ersten Katheters (11) ist.

## Claims

1. A device for introducing a tissue transplant (2, 103) into a hollow organ (4) comprising a support (21, 102) having a surface for supporting the tissue transplant (2, 103) and a protective element (11, 104) for protecting the tissue transplant (2, 103) while the device (1, 101) being introduced into the hollow organ (4) **characterized in that** the device comprises:
- a first tube (11) that serves as a protective element and has an open proximal end (12);
- a second tube (21) that serves as a support and can be inserted into the first tube (11) and has a first portion (23) having the surface (24) for supporting the tissue transplant (2) and being able to be expanded by supplying a fluid; and
- a third tube (31) that can be guided through the second tube (21) and the open proximal end (12) of the first tube (11), wherein the third tube (31) at its proximal end has a second portion (32) being able to be expanded by supplying a fluid.

2. The device according to claim 1 **characterized in that** the tissue transplant (2, 103) is an implant.

3. The device according to any of the preceding claims **characterized in that** the tissue transplant (2, 103) is selected from the group comprising skin; oral mucosa; native tissue, synthetic tissue; a membrane; a membrane having cells and/or cell constituents; a membrane having no cells and no cell constituents, a membrane having a treated surface as well as a membrane composite.

4. The device according to claim 3 **characterized in that** the membrane or the membrane composite is populated with mucosa tissue cells.

5. The device according to any of the preceding claims **characterized in that** the longitudinal axes of the first, second, and third tubes (11, 21, 31) run coaxial to each other.

6. The device according to any of the preceding claims **characterized in that** it further has attachment means for detachably attaching the tissue transplant (2) to the surface (24) of the first expandable portion (23).

7. The device according to any of the preceding claims **characterized in that** the second expandable portion (32) of the third tube (31) has a maximum expansion transverse to the longitudinal axis (A) of the third tube (31) that corresponds to the expansion of the first tube (11) transverse to its longitudinal axis (A) or exceeds its expansion.

8. The device according to any of the preceding claims **characterized in that** the second expandable portion (32) of the third tube (31) in the expanded state has an expansion transverse to its longitudinal axis (A) tapering in the proximal direction.

9. A method for the preparation of a device according to any of claims 1 to 8 **characterized in that** it comprises the steps of:
(a) applying the tissue transplant (2) onto the surface (24) for receiving the tissue transplant (2) that is formed on the first expandable portion (23) of the second tube (21);
(b) inserting the second tube (21) into the first tube (11); and
(c) guiding the third tube through the second tube and the open proximal end (12) of the first tube (11).

10. The device according to any of claims 1 to 8 **characterized in that** the device is an assembly comprising a support (21, 102) having a surface for supporting the tissue transplant (2, 103) and a protective element (11, 104) for protecting the tissue transplant while the assembly being introduced into the hollow organ (4), wherein:
- the first tube serving as a protective element is a first catheter (11) having an open proximal end (12);
- the second tube serving as a support is a second catheter (21) for insertion into the first catheter (11), wherein the second catheter (21) has a first portion (23) having a surface (24) for receiving the tissue transplant (2) and being able to be expanded by supplying a fluid; and
- the third tube is a third catheter (31) for guiding through the second catheter (21) and the open proximal end (12) of the first catheter (11).

## Revendications

1. Dispositif pour introduire un ersatz de tissu (2, 103) dans un organe creux (4), comprenant un support (21, 102) avec une surface pour porter l'ersatz de tissu (2, 103) et un élément de protection (11, 104) pour protéger l'ersatz de tissu (2, 103) durant l'introduction du dispositif (1, 101) dans l'organe creux (4), **caractérisé par le fait que** ce dispositif comprend :
- un premier tube (11) servant d'élément de protection, avec une extrémité proximale (12) ouverte ;
- un deuxième tube (21) servant de support qui est introduisible dans le premier tube (11) et présente une première section (23), expansible par l'apport d'un fluide, avec la surface (24) pour porter l'ersatz de tissu (2), et
- un troisième tube (31) qui est introduisible dans le deuxième tube (21) et par l'ouverture proximale ouverte (12) du premier tube (11), lequel troisième tube (31) présente à son extrémité proximale une section (32) expansible par l'apport d'un fluide.

2. Dispositif selon la revendication 1 **caractérisé par le fait que** l'ersatz de tissu (2, 103) est un implant.

3. Dispositif selon une des revendications précédentes **caractérisé par le fait que** l'ersatz de tissu (2, 103) est choisi parmi un groupe qui comprend peau, muqueuse buccale, cellules souches, cellules obtenues artificiellement, une membrane, une membrane présentant des cellules et/ou des éléments de cellule, une membrane qui ne comprend ni cellules ni éléments de cellule, une membrane à surface traitée ainsi qu'une combinaison de membranes.

4. Dispositif selon la revendication 3 **caractérisée par le fait que** la membrane ou la combinaison de membranes est colonisée par des cellules de tissu muqueux.

5. Dispositif selon une des revendications précédentes **caractérisé par le fait que** les axes longitudinaux des premier, deuxième et troisième tubes (11, 21, 31) s'étendent coaxialement entre eux.

6. Dispositif selon une des revendications précédentes **caractérisé par le fait qu'**il présente en outre, à la surface (24) de la première section expansible (23), des moyens de maintien et de libération de l'ersatz de tissu (2).

7. Dispositif selon une des revendications précédentes **caractérisé par le fait que** la deuxième section expansible (32) du troisième tube (31) comprend un élargissement maximum transversal à l'axe longitudinal (A) du troisième tube (31) qui correspond à l'élargissement du premier tube (11) transversal à l'axe longitudinal (A) de celui-ci, ou dépasse cet élargissement.

8. Dispositif selon une des revendications précédentes **caractérisé par le fait que** la deuxième section expansible (32) du troisième tube (31) présente en position expansée un rétrécissement en direction proximale transversalement à l'axe longitudinal (A).

9. Procédé pour la fabrication d'un dispositif selon une des revendications 1 à 8 **caractérisé par le fait qu'**il comprend les étapes suivantes :
(a) application de l'ersatz de tissu (2) sur la surface (24) de réception de l'ersatz de tissu (2) formée à la première section (23) expansible du deuxième tube (21) ;
(b) introduction du deuxième tube (21) dans le premier tube (11), et
(c) guidage du troisième tube à travers le deuxième tube et à travers l'ouverture proximale ouverte (12) du premier tube (11) .

10. Dispositif selon une des revendications précédentes 1 à 8 **caractérisé par le fait que** ledit procédé est une disposition qui comprend un support (21, 102) avec une surface apte à porter l'ersatz de tissu (2, 103) et un élément de protection (11, 104) pour la protection de l'ersatz de tissu durant l'introduction de cette disposition dans l'organe creux (4), étant donné que :
- le premier tube servant d'élément de protection est un premier cathéter (11) avec une extrémité proximale ouverte (12) ;
- le deuxième tube servant de support est un deuxième cathéter (21) pour l'introduction dans le premier cathéter (11), étant donné que ledit deuxième cathéter (21) présente une première section, expansible par l'apport d'un fluide, avec une surface (24) pour recevoir l'ersatz de tissu (2) ; et
- le troisième tube est un troisième cathéter (31) de guidage dans le deuxième cathéter (21) et par l'extrémité proximale ouverte (12) du premier cathéter (11).
